# EUROPEAN PATENT SPECIFICATION

(11) **EP 2 213 969 B1**
(45) Date of publication and mention of the grant of the patent: **01.07.2020**
(21) Application number: 08849406.7
(22) Date of filing: 03.10.2008
(51) Int. Cl.: F25D 23/00, A61L 9/22, F25D 17/04

(54) **REFRIGERATOR**
KÜHLSCHRANK
RÉFRIGÉRATEUR

(30) Priority: 16.11.2007 JP 2007297899
(43) Date of publication of application: 04.08.2010
(73) Proprietor: Sharp Kabushiki Kaisha, Osaka 590-8522 (JP)
(72) Inventor: The designation of the inventor has not yet been filed
(74) Representative: Treeby, Philip David William
(86) International application number: PCT/JP2008/068038
(87) International publication number: WO 2009/063707

(56) References cited:
- EP-A1- 1 348 923
- EP-A2- 1 806 552
- JP-A- 1 219 480
- JP-A- 2002 333 265
- JP-A- 2003 014 365
- JP-U- 60 069 977

## Description

### Technical Field

The present invention relates to a refrigerator provided with an ion generator that generates ions.

### Background Art

A conventional refrigerator having an ion generator that generates ions is disclosed in Patent Document 1. This refrigerator has a cool air duct arranged behind a refrigerator compartment. In the cool air duct, a plurality of discharge ports are open to face the refrigerator compartment, and near a first discharge port, an ion generator is arranged.

As the ion generator is driven, minus ions are released, along with cool air flowing through the cool air duct, through the discharge ports into the refrigerator compartment. This permits removal of odor components of articles-to-be-stored. Moreover, by generating minus ions and plus ions by the ion generator and blowing them to a storage compartment, it is possible to sterilize inside the storage compartment.

Patent Document 1: JP-A-2003-14365 (pages 2-6, Fig. 3)

JP 60 069977 U discloses a refrigerator according to the preamble of claim 1.

EP 1,806,552 discloses a refrigerator wherein an ion generator is arranged in a cold air duct which supplies cold air to a storage compartment, and a desired portion of the cold air duct is treated for anti-static. The ions from the ion generator are used for the purpose of sterilization and deodorization and are effectively discharged into the storage compartment. The anti-static-treatment of the cold air duct prevents the ions generated from the ion generator from being lost due to static in the cold air duct. The anti-static treatment is carried out by coating polyethylene on a portion of the cold air duct. The cold air duct includes a plurality of uniformly spaced cold air outlet, whereby the ions generated from the ion generator are uniformly distributed into the overall portion of the storage compartment through the cold air outlets.

EP 1,348,923 discloses a storage unit and refrigerator wherein the air inside a refrigerator compartment is introduced through a return opening into an ion generation chamber provided behind the refrigerator compartment. A voltage is applied to a needle-like electrode arranged in an upper portion of the ion generation chamber so that, by corona discharge, positive and negative ions are discharge substantially parallel to the cool air circulating in the direction indicated by arrow B2. This helps alleviate the loss of ions resulting from collision with a wall surface, and permits the ions to reach a wide area. This prolongs the period for which the cool air is kept in contact with the ions, and thus helps enhance the sterilizing effect.

### Disclosure of the Invention

### Problems to be Solved by the Invention

An object of the present invention is to provide a refrigerator that enhances sterilizing effect.

### Means for Solving the Problem

To achieve the above object, according to the present invention, there is provided a refrigerator as defined in claim 1.

With this structure, cool air generated by the cooler flows into the cool air passage arranged at the rear face of the storage compartment, and includes ions generated by the ion generator. The cool air including the ions branches into the first and second branched passages, and flows, as it spreads out, along the rear face of the storage compartment, and is discharged through the discharge port into the storage compartment.

Moreover, according to the invention, in the refrigerator structured as described above, the cool air passage comprises a flow passage widened at the downstream of where cool air flows into the cool air passage from the cooler, and the ion generator is provided at the upstream of where the cool air passage widens. With this structure, cool air that has flowed into the cool air passage passes through a narrow passage and includes ions thanks to the provision of the ion generator. The cool air that has included the ions flows, as it spreads out, through the widened passage with its flow rate lowered, and branches into the first and second branched passages to be discharged gently through the discharge port into the storage compartment.

Moreover, according to a preferred embodiment of the invention, in the refrigerator structured as described above, a part, into which cool air flows, of the cool air passage is provided lopsidedly in one of left and right directions, and the longitudinal direction of the ion generator is inclined such that a downstream end thereof points to another of the left and right directions. With this structure, cool air flows into the cool air passage at a position lopsided toward the first branched passage. The cool air flowed into the cool air passage is guided by the ion generator of which the longitudinal direction extends toward the second branched passage, and cool air including ions is led to the second branched passage.

Moreover, according to a preferred embodiment of the invention, in the refrigerator structured as described above, the ion generator is arranged lopsidedly in a direction perpendicular to a direction in which cool air flows, with an electrode face that generates ions aligned along a direction in which cool air flows and with a face opposite from the electrode face being apart from a wall face of the cool air passage. With this structure, ions generated from the electrode face of the ion generator are included in the cool air flowing along the electrode face. In addition, the cool air flowing along the face opposite from the electrode face mixes with the cool air including the ions.

Moreover, according to a preferred embodiment of the invention, in the refrigerator structured as described above, the the refrigerator is configured so that the temperature of cool air that is in contact with the ion generator is higher than - 20 °C.

Moreover, according to a preferred embodiment of the invention, in the refrigerator structured as described above, the storage compartment comprises a refrigerator compartment, the storage compartment being provided with a separated compartment with a temperature lower than refrigeration temperature, and the ion generator is arranged at the upstream of a discharge port of the separated compartment. With this structure, the cool air that has flowed into the cool air passage includes ions thanks to the provision of the ion generator. The cool air including the ions is discharged to the separated compartment, comprising a chilled compartment or the like, through the discharge port.

### Advantages of the Invention

According to the present invention, since an ion generator is provided at the upstream of where a first and second branched passages branch, cool air including ions flows, as it spreads out, along the rear face of a storage compartment to be discharged through a discharge port. Accordingly, the ion concentration in the storage compartment is made even, and the sterilizing effect is improved.

Moreover, according to the invention, since the flow passage of the cool air passage is widened at the downstream of where cool air flows thereinto, the flow rate of the cool air is lowered and disappearance of ions due to collision is reduced. Moreover, since the ion generator is provided at the upstream of where the cool air passage widens, the cool air includes ions before they are diffused. Accordingly, the cool air flowing through the cool air passage surely includes the ions, further improving the sterilizing effect.

Moreover, according to the invention, since a part, into which cool air flows, of the cool air passage is provided lopsidedly toward one of the first and second branched passages, on the right and left sides respectively, and the longitudinal direction of the ion generator is so arranged that a downstream end points in another of the first and second branched passages, the ion generator functions as a wind deflecting plate. Thus, it is possible to lead cool air to a part away from the part, into which cool air flows, of the first and second branched passages. Accordingly, cool air flows evenly through the first and second branched passages, and the ion concentration in the storage compartment is made more even.

Moreover, according to the invention, since an electrode face of the ion generator is aligned along the direction in which cool air flows, cool air includes ions easily. Moreover, since the ion generator is arranged lopsidedly in the flow passage with a face thereof opposite from the electrode face being apart from a wall face of the cool air passage, cool air flowing though a part away from the ion generator includes ions easily. In addition, cool air flowing along the face opposite from the electrode face mixes with the cool air flowing along the electrode face to thereby include ions. Accordingly, the cool air flowing through the cool air passage evenly includes ions, and the ion concentration in the storage compartment is made more even.

Moreover, according to the invention, since the temperature of cool air which is in contact with the ion generator is higher than - 20 °C, it is possible to reduce accumulation of frost on an electrode of the ion generator. In a case of an ion generator in which a high voltage generator that applies a high voltage to the electrode and the electrode unitized, electrical components in the high voltage generator are maintained at a temperature higher than - 20 °C. This helps prevent failure of maintaining discharging due to the temperature of the electrical components in the high voltage generator falling to - 20 °C or below. Accordingly, the electrode surely discharges and generates ions.

Moreover, according to the invention, since the ion generator is arranged at the upstream of a discharge port of a separated compartment with a temperature lower than refrigeration temperature, it is possible to sterilize inside the separated compartment such as a chilled compartment.

### Brief Description of Drawings

[Fig. 1] A front view showing a refrigerator according to a first embodiment of the present invention
[Fig. 2] A right side view showing the refrigerator according to the first embodiment of the invention.
[Fig. 3] A right side sectional view showing the refrigerator according to the first embodiment of the invention.
[Fig. 4] A front view showing a main unit of the refrigerator according to the first embodiment of the invention.
[Fig. 5] A front view showing a panel assembly of the refrigerator according to the first embodiment of the invention.
[Fig. 6] A rear view showing the panel assembly of the refrigerator according to the first embodiment of the invention.
[Fig. 7] A front view showing a panel assembly of a refrigerator according to a second embodiment of the invention.
[Fig. 8] A rear view showing the panel assembly of the refrigerator according to the second embodiment of the invention.
[Fig. 9] A front view showing a panel assembly of a refrigerator according to a third embodiment of the invention.
[Fig. 10] A rear view showing the panel assembly of the refrigerator according to the third embodiment of the invention.

### List of Reference Symbols

- 1: refrigerator
- 2: refrigerator compartment
- 3: temperature switching compartment
- 4: ice-making compartment
- 5: vegetable compartment
- 6: freezer compartment
- 7, 8, 35: heat-insulating wall
- 11: cooler
- 12: freezer-compartment blower
- 15: lead-in air flow passage
- 16: heater
- 17: return air flow passage
- 18: temperature-switching-compartment blower
- 20: refrigerator-compartment damper
- 22: freezer-compartment return port
- 23: refrigerator-compartment blower
- 31, 32: cool air passage
- 32a: first branched passage
- 32b: second branched passage
- 32c: inlet portion
- 32d: widened portion
- 36: vertical heat-insulating wall
- 37: temperature-switching-compartment discharge damper
- 38: temperature-switching-compartment return damper
- 57: compressor
- 100: panel
- 101: member
- 102: sealing member
- 102a: project portion
- 103a, 103b, 104a, 104b, 105a, 105b: discharge port
- 108: tank
- 111: space portion
- 120: panel assembly
- 130: ion generator
- 130a: electrode face

### Best Mode for Carrying Out the Invention

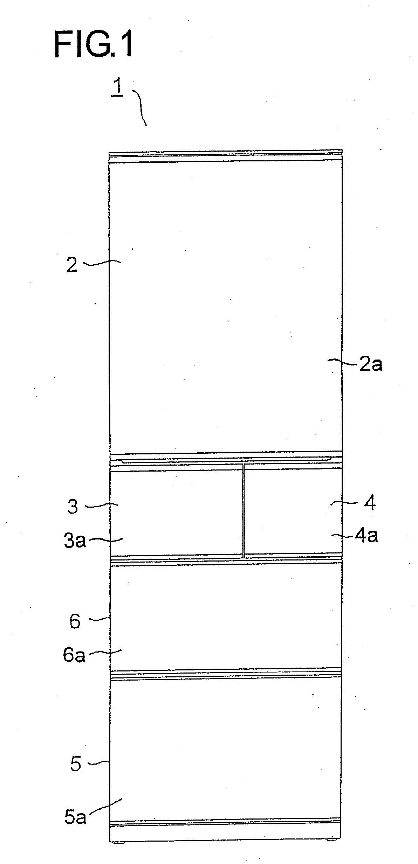

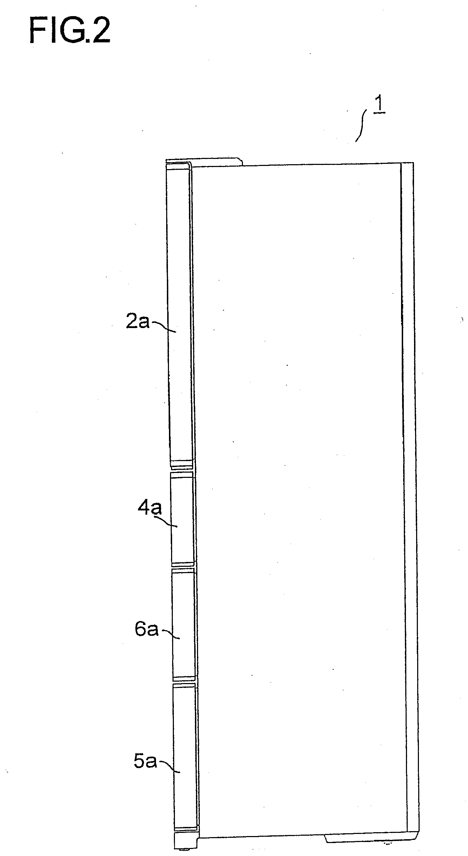

Embodiments of the present invention will be described below with reference to the relevant drawings. Fig. 1 and Fig. 2 are a front view and a right side view, respectively, showing a refrigerator according to a first embodiment of the invention. In an upper part of the refrigerator 1, a refrigerator compartment 2 (a storage compartment) opened/closed by a door 2a is arranged. Below the refrigerator compartment 2, a temperature switching compartment 3 and an ice-making compartment 4 opened/closed by a door 3a and a door 4a, respectively, are provided side by side on the left and right. Below the temperature switching compartment 3 and the ice-making compartment 4, a freezer compartment 6 opened/closed by a door 6a is arranged, and below the freezer compartment 6, a vegetable compartment 5 opened/closed by a door 5a is arranged.

The refrigerator compartment 2 refrigerates to preserve articles-to-be-stored, and the vegetable compartment 5 cools to preserve vegetables at a room temperature (about 8 °C), which is higher than the temperature in the refrigerator compartment 2. As the details will be described later, the temperature in the temperature switching compartment 3 can be switched by the user. The freezer compartment 6 freezes to preserve articles-to-be-stored, and the ice-making compartment 4 communicates with the freezer compartment 6 and makes ices. The ice-making compartment 4 and the freezer compartment 6 are maintained at or below freezing point, and, in this specification, the ice-making compartment 4 forms part of the freezer compartment 6.

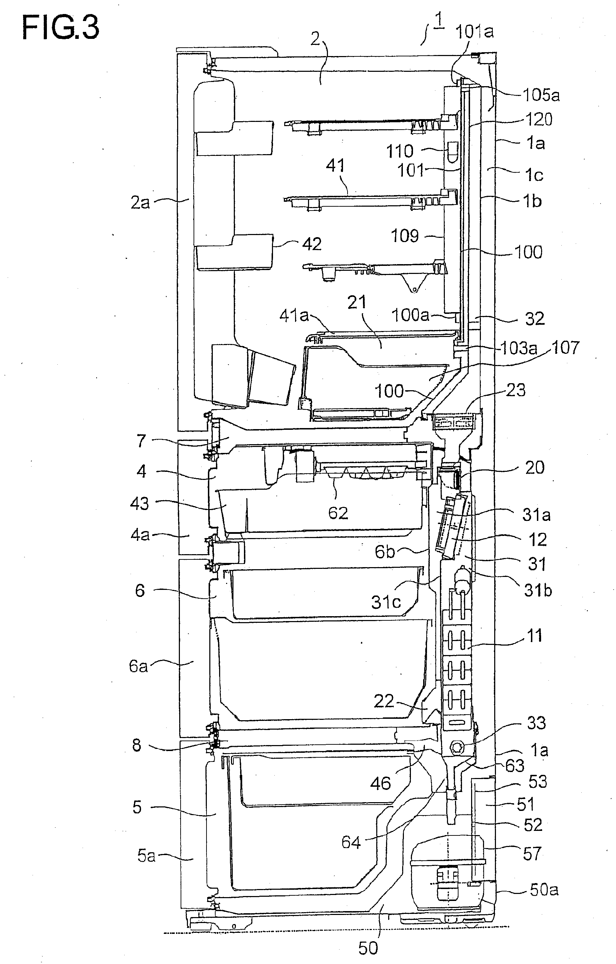

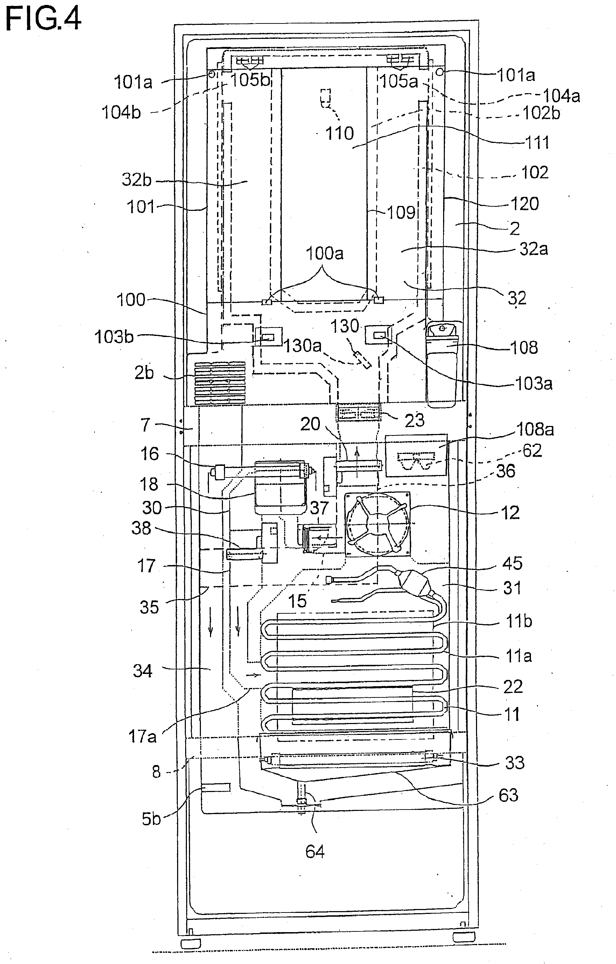

Fig. 3 and Fig. 4 are a right side sectional view of the refrigerator 1 and a front view of a main unit of the refrigerator 1, respectively. The main unit of the refrigerator 1 has a foam heat-insulating material 1c filled between an outer case 1a and an inner case 1b. The ice-making compartment 4 and the temperature switching compartment 3 are separated from the refrigerator compartment 2 by a heat-insulating wall 7, and the freezer compartment 6 is separated from the vegetable compartment 5 by a heat-insulating wall 8. In addition, the temperature switching compartment 3 is separated from the freezer compartment 6 by a heat-insulating wall 35, and the temperature switching compartment 3 is separated from the ice-making compartment 4 by a vertical heat-insulating wall 36.

The foam heat-insulating material 1c is formed of an urethane foam heat-insulating material or the like, and, when the foam heat-insulating material 1c is filled between the outer case 1a and the inner case 1b, it is filled inside the heat-insulating walls 7 and 8 simultaneously. Specifically, a stock solution of the foam heat-insulating material 1c is filled in a part between the outer case 1a and the inner case 1b and, simultaneously, inside the heat-insulating walls 7 and 8 that are communicates with the part, and is foamed as a whole. This permits easy formation of thin heat-insulating walls 7 and 8. Accordingly, a large volume of the refrigerator compartment 2 is obtained.

The exterior of the heat-insulating walls 7 and 8 comprises the inner case 1b and another member, and, before the foam heat-insulating material 1c is filled, side faces of the heat-insulating walls 7 and 8 are open, and the inner case 1b is open to face those side faces of the heat-insulating walls 7 and 8. With the foam heat-insulating material 1c filled in, the openings of the side faces of the heat-insulating walls 7 and 8 and the opening of the inner case 1b are connected together to be integral.

This prevents leakage of cool air or hot air between different storage compartments - with different temperature zones - that are separated by the heat-insulating walls 7 and 8. Accordingly, energy is saved by reducing heat loss. Moreover, abnormal noise is prevented that is caused by the heat-insulating walls 7 and 8 vibrating, and the heat-insulating wall 7 or 8 rubbing against the inner case 1b due to the vibration. In addition, the structural strength is enhanced by integral formation.

The ice-making compartment 4, the freezer compartment 6, the vegetable compartment 5, and the temperature switching compartment 3 are each provided with a storage case 43 for storing articles-to-be-stored. The refrigerator compartment 2 is provided with a plurality of storage shelves 41 for placing articles-to-be-stored. At the door 2a of the refrigerator compartment 2, a plurality of storage pockets 42 are provided. These improve user friendliness of the refrigerator 1.

In a lower part inside the refrigerator compartment 2, a chilled compartment 21 is provided that is formed of a separated compartment having the top face thereof partitioned by a partition plate 41a to be separated. The chilled compartment 21 is maintained at a temperature lower than those of other regions, for example, at a chilled temperature zone (about 0 °C). In the chilled compartment 21, a storage case 107 for storing articles-to-be-stored is arranged. The chilled compartment 21 may be switched to an ice-temperature compartment that is maintained at an ice temperature (about - 3 °C).

Behind the vegetable compartment 5, a machine compartment 50 is provided, and thereinside, a compressor 57 is arranged. To the compressor 57, a condenser, an expander (neither of them is illustrated), and a cooler 11 are connected, and, as the compressor 57 is driven, a refrigerant such as isobutane circulates to operate a refrigerating cycle. Thus, the cooler 11 serves as a cold side of the refrigerating cycle.

In a rear part of the machine compartment 50, an electrical component portion 51 that includes electrical components such as a control circuit board 53 is provided. The electrical component portion 51 has an electrical component box 52 whose rear face - fitted to the main unit of the refrigerator 1 - is open and is hermetically sealed by a rear face cover 50a for covering the rear face of the machine compartment 50. The electrical component box 52 is formed by drawing a metal plate and, with a large heat dissipating area, dissipates heat generated from the electrical components easily and hermetically seals the electrical component portion 51 easily.

Behind the freezer compartment 6, a cool air passage 31 partitioned by a rear face plate 6b is provided. Behind the refrigerator compartment 2, a cool air passage 32 that communicates with the cool air passage 31 via a refrigerator-compartment damper 20 is provided. The cool air passage 31 is partitioned into a front part 31a and a rear part 31b by a partition plate 31c, and the cooler 11 is arranged in the rear part 31b. Since the cooler 11 is arranged at the rear face of the freezer compartment 6, cold of the cooler 11 is released toward the freezer compartment 6 via the partition plate 31c, the front part 31a, and the rear face plate 6b. Thus, the freezer compartment 6 is indirectly cooled efficiently, and the cooling efficiency is improved.

The cooler 11 serving as the cold side of the refrigerating cycle exchanges heat with the air flowing through the cool air passage 31 to generate cool air. Below the cooler 11, a defrost heater 33 that defrosts the cooler 11 is provided. Below the defrost heater 33, a drain pan 63 that receives defrost water is provided. The drain pan 63 is provided with a drain pipe 64, and drained water is led, via the drain pipe 64, to an evaporation plate (unillustrated) arranged in the machine compartment 50.

In the cool air passage 31 and the cool air passage 32, a freezer-compartment blower 12 and a refrigerator-compartment blower 23 are arranged respectively. As details will be described later, by driving of the freezer-compartment blower 12, cool air generated by the cooler 11 flows through the front part 31a of the cool air passage 31 to be fed to the freezer compartment 6, the ice-making compartment 4, and the temperature switching compartment 3. In addition, by driving of the refrigerator-compartment blower 23, the cool air is fed, through the cool air passage 32, to the refrigerator compartment 2, the chilled compartment 21, and the vegetable compartment 5.

The freezer-compartment blower 12 comprises an axial-flow fan, and is arranged with a discharge side facing front-upward. Thus, the cool air cooled by the cooler 11 located below is efficiently sucked from obliquely rearward of the freezer-compartment blower 12. In addition, cool air is blown out front upward toward the front part 31a of the cool air passage 31 to be discharged into the ice-making compartment 4 and to be led to the cool air passage 32 located above. Accordingly, it is possible to let cool air flow upward efficiently, and to reduce noise and save energy.

The refrigerator-compartment blower 23 comprises an axial-flow fan, and is arranged with the axis direction aligned along the up/down direction. Thus, as described above, it is possible to let cool air flow upward efficiently, and to reduce noise and save energy. In addition, the refrigerator-compartment blower 23 is lowered in the height direction, and the refrigerator-compartment blower 23 and the heat-insulating wall 7 are arranged in a single horizontal plane so that they overlap in a front projection.

Thus, no refrigerator-compartment blower 23 is arranged behind the refrigerator compartment 2 which is used frequently, making the depth of the cool air passage 32 narrow. Specifically, the cool air passage 32 is formed to have a depth of, for example, 80 mm on the discharge side of the refrigerator-compartment blower 23, and is formed to be increasingly narrow toward a downstream part of air flow so as to be, for example, 12 mm.

Here, the total width in the left/right direction of the cool air passage 32 is formed wider than near the discharge side of the refrigerator-compartment blower 23. This secures the air passage area of the cool air passage 32 so that the amount of cool air flowing is maintained and lowered blowing efficiency is prevented. Accordingly, the depth of the refrigerator compartment 2 in front of the narrowed cool air passage 32 increases, and a large capacity of the refrigerator compartment 2 is obtained.

Moreover, since the refrigerator-compartment blower 23 is provided in a region overlapping the heat-insulating wall 7 in the up/down direction, the freezer-compartment blower 12 is arranged in a place lower than and away from an ice-making plate 62 which is arranged in an upper part of the ice-making compartment 4. However, since the discharge direction of cool air of the freezer-compartment blower 12 is in the direction toward the ice-making plate 62 to the above front, water stored in the ice-making plate 62 is cooled efficiently.

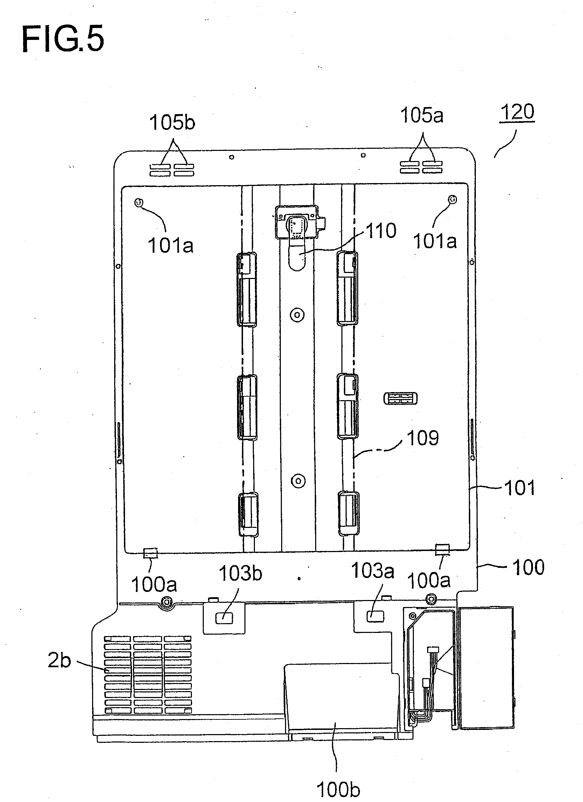

At the downstream of the refrigerator-compartment blower 23, the cool air passage 32 has the front face thereof covered by a panel assembly 120. Fig. 5 shows a front view of the panel assembly 120. The panel assembly 120 has a member 101 having high thermal conductivity arranged on the front face of a panel 100 comprising a resin molded component. For the panel 100, resin such as PP, PS, and ABS that is easy to process is used. For the member 101, metal such as aluminum and highly anticorrosive stainless steel is used.

The member 101 is detachably provided in an upper part of the panel 100 by engagement claws 100a that project from the panel 100, and fitting members 101a. Accordingly, it is possible to clean the member 101 easily, and to maintain inside of the refrigerator compartment 2 clean. The panel assembly 120 is fitted to the inner case 1b with engagement claws (unillustrated) provided in a circumference part of the rear face of the panel 100. For the fitting members 101a, screws, push pins formed of resin, of which the tips are arrowhead-like, or the like are used.

In a central upper part of the front face of the member 101, an illumination lamp 110 that is covered by a lamp cover 109 is arranged. Light emitted from the illumination lamp 110 illuminates inside the refrigerator compartment 2 from above, and is reflected at the surface of the member 101 to illuminate inside the refrigerator compartment 2 from behind. Thus, inside the refrigerator compartment 2 is made even brighter. Desirably, the lamp cover 109 and the storage shelves 41 are formed of opaque or transparent glass or resin (such as ABS, PS, polycarbonate, and acrylic).

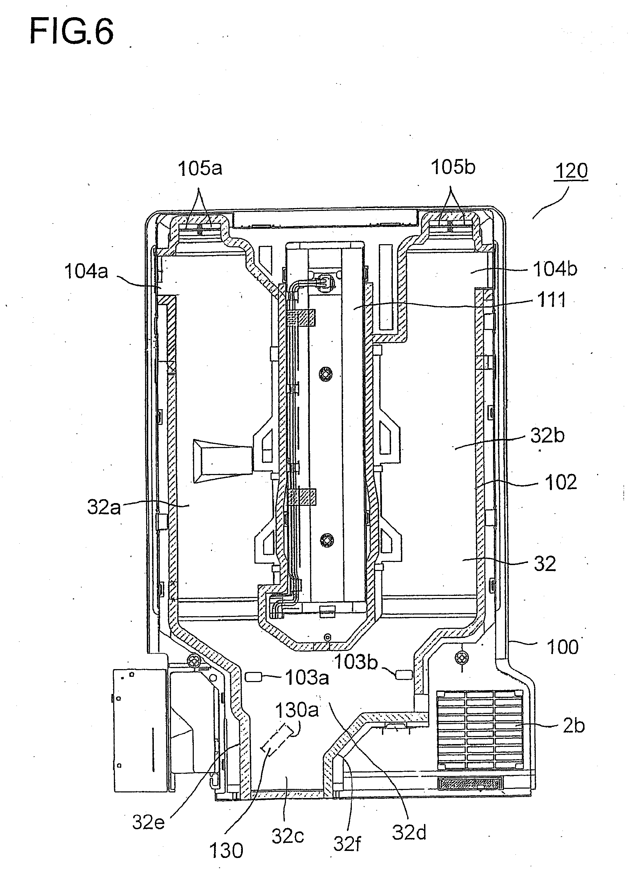

In a lower left part of the panel 100, a refrigerator-compartment outlet port 2b is formed, through which cool air flows out from the refrigerator compartment 2. In a position lopsidedly rightward in a lower part of the panel 100, a project portion 100b projecting frontward is provided. The project portion 100b has the refrigerator-compartment blower 23 arranged directly below it, so that an inlet portion 32c (see Fig. 6) into which cool air flows is formed in a part, at the back of the refrigerator compartment 2, of the cool air passage 32.

Fig. 6 shows a rear view of the panel assembly 120. From the rear face of the panel 100, a rib (unillustrated) projects, and, on this rib, a sealing member 102 is affixed. The sealing member 102 comprises an elastic member, which keeps contact with the inner case 1b to form the cool air passage 32 between the panel 100 and the inner case 1b. The cool air passage 32 provided above the refrigerator-compartment damper 20 has the inlet portion 32c, of which the width of the passage is narrow, in a lower part of the panel assembly 120.

As the refrigerator-compartment damper 20 is opened, the cool air flowing into the cool air passage 32 is led via the inlet portion 32c to an upper part of the refrigerator compartment 2. The refrigerator-compartment damper 20 and the inlet portion 32c are provided lopsidedly on the right side (the left side in Fig. 6) where the ice-making compartment 4 is arranged. Thus, cold of the cool air is released via the cool air passage 32 to the ice-making compartment 4 at or below the freezing point, and thus no cold is drawn by the temperature switching compartment 3 with high temperature, and the cooling efficiency of the refrigerator 1 is improved.

The cool air passage 32 has a widened portion 32d where the flow passage is widened at the downstream of the inlet portion 32c, and is provided with a first and second branched passages 32a and 32b branching from the widened portion 32d. Between the first and second branched passages 32a and 32b, a space portion 111 is formed. In the widened portion 32d, discharge ports 103a and 103b for discharging cool air to the chilled compartment 21 are open in the panel 100.

The first branched passage 32a is provided to extend substantially above the inlet portion 32c. The second branched passage 32b is arranged via the widened portion 32d to the left (to the right in Fig. 6) of the inlet portion 32c and extends upward. In upper parts of the first and second branched passages 32a and 32b, discharge ports 104a, 104b, 105a, and 105b for discharging cool air are formed. The discharge ports 104a and 104b discharge the cool air sideward from an upper part of the member 101. The discharge ports 105a and 105b are arranged above the member 101 and discharges the cool air frontward.

In the inlet portion 32c of the cool air passage 32, an ion generator 130 is arranged lopsidedly on the right side (the left side in Fig. 6). The ion generator 130 is formed substantially rectangular as seen in a plan view, and is arranged with an electrode face 130a that has an electrode (unillustrated) facing a side wall 32f of the inlet portion 32a, which is on the farther side. This allows the electrode face 130a to be provided along the cool air rising through the inlet portion 32c.

To the electrode of the electrode face 130a, a voltage comprising an alternating waveform or an impulse waveform is applied. With respect to the voltage applied to the electrode face 130a, in a case where it is a positive voltage, plus ions comprising mainly H⁺(H₂O)ₙ are generated; in a case where it is a negative voltage, minus ions comprising mainly O₂⁻(H₂O)ₘ are generated. H⁺(H₂O)ₙ and O₂⁻(H₂O)ₘ aggregate on the surfaces of microorganisms to capture floating bacteria of microorganisms etc. in the air. Here, n and m are integers.

As shown in formulae (1) to (3), [·OH] (hydroxyl radical) or H₂O₂ (hydrogen peroxide) - an active species by collision - is aggregated and generated on the surfaces of microorganisms or the like to perform sterilization of floating bacteria. Accordingly, the plus ions and minus ions generated from the electrode face 130a are included in the cool air flowing through the inlet portion 32c, permitting sterilization of different storage compartments of the refrigerator 1. Note that n' and m' are integers.

H⁺(H₂O)ₙ + O₂⁻(H₂O)ₘ → · OH + 1/2 O₂ + (n + m) H₂O ··· (1)

H⁺(H₂O)ₙ + H⁺(H₂O)_{n'} + O₂⁻(H₂O)ₘ + O₂⁻(H₂O)_{m'} → 2 · OH + O₂ + (n + n' + m + m') H₂O ··· (2)

H⁺(H₂O)ₙ + H⁺(H₂O)_{n'} + O₂⁻(H₂O)ₘ + O₂⁻(H₂O)_{m'} → H₂O₂ + O₂ + (n + n' + m + m') H₂O ··· (3)

The ion generator 130 is arranged with a face thereof opposite from the electrode face 130a being apart from a side wall 32e of the inlet portion 32c, which is on the closer side. Since the ion generator 130 generates ions from the electrode face 130a provided on one side, the electrode face 130a needs to be arranged with it facing the cool air flowing through the inlet portion 32c. Since the inlet portion 32c has a narrow passage width, it is easy for the whole cool air that flows through the inlet portion 32c to include ions.

Furthermore, since the face opposite from the electrode face 130a is arranged apart from the wall face 32e of the inlet portion 32c, ions reach the proximity of the side wall 32f away from the ion generator 130. Here, although no ion is included in the cool air flowing between the side wall 32e and the ion generator 130, the cool air mixes with the cool air, that flows along the electrode face 130a, above the ion generator 130 and includes ions. This helps the whole cool air flowing through the inlet portion 32c surely include ions.

The longitudinal direction of the ion generator 130 which is long in the up/down direction is arranged to incline such that a downstream part of cool air heads for the second branched passage 32b. The cool air flowing through the inlet portion 32c provided lopsidedly on the right side tends to flow into the first branched passage 32a on the right side. Thus, by arranging the ion generator 130 along a direction that points to the second branched passage 32b, it is possible to guide the cool air toward the second branched passage 32b. Accordingly, the ion generator 130 functions as a wind deflecting plate, permitting the cool air to flow evenly through the first and second branched passages 32a and 32b.

The cool air that has included ions at the inlet portion 32c flows through the widened portion 32d and is discharged through the discharge ports 103a and 103b into the chilled compartment 21 to sterilize there. Also, the cool air flows through the first and second branched passages 32a and 32b, and is discharged through the discharge ports 104a, 104b, 105a, and 105b into the refrigerator compartment 2. Thus, the refrigerator compartment 2 and the vegetable compartment 5 communicating with the refrigerator compartment 2 are sterilized.

In Fig. 4, in the refrigerator compartment 2, a tank 108 for automatic ice making is arranged, which is separated from the chilled compartment 21 (see Fig. 3) by a separation wall (unillustrated). Water in the tank 108 passes through a pipe (unillustrated) by a pump (unillustrated), and is fed to the ice-making plate 62 of an ice-making device 108a provided in the ice-making compartment 4 located below. In this way, water is automatically fed to the ice-making plate 62 and ice is made automatically.

The refrigerator-compartment blower 23, the refrigerator-compartment damper 20, and the freezer-compartment blower 12 are arranged approximately one above another in the up/down direction. Specifically, the refrigerator-compartment blower 23, the refrigerator-compartment damper 20, and the freezer-compartment blower 12 are arranged such that they overlap in a plane projection. Thus, the width of the refrigerator 1 in the left/right direction is made narrow, and the cool air passages 31 and 32 are shortened, further improving the volume efficiency and the blowing efficiency.

Behind the freezer compartment 6, the cool air passage 31 has a part of its face in front of the freezer-compartment blower 12 open, and, by the freezer-compartment blower 12, air is blown into the ice-making compartment 4. In a lower part of the freezer compartment 6 that communicates with the ice-making compartment 4, a freezer-compartment return port 22 is provided. In addition, a lead-in air flow passage 15 is provided that branches from the cool air passage 31 to lead cool air into the temperature switching compartment 3.

An upper part of the cool air passage 31 communicates with the cool air passage 32 via the refrigerator-compartment damper 20. As the refrigerator-compartment damper 20 is opened and the freezer-compartment blower 12 is driven, cool air is fed to the refrigerator compartment 2 and the chilled compartment 21. The refrigerator-compartment damper 20 is arranged at the back of the vertical heat-insulating wall 36 such that it overlaps with the vertical heat-insulating wall 36 in a front projection.

In a lower part of the rear face of the refrigerator compartment 2, the refrigerator-compartment outlet port 2b is open. In the vegetable compartment 5, a vegetable-compartment inlet port 5b is provided. The refrigerator-compartment outlet port 2b is connected with the vegetable-compartment inlet port 5b by a connection passage 34 that passes along the rear face of the temperature switching compartment 3, and thereby the refrigerator compartment 2 communicates with the vegetable compartment 5. In an upper rear part of the vegetable compartment 5, a return air flow passage 46 (see Fig. 3) communicating with the cool air passage 31 is provided.

In an upper part of the temperature switching compartment 3, a temperature-switching-compartment blower 18 and a heater 16 are arranged. In a lower right part of the temperature switching compartment 3, a temperature-switching-compartment discharge damper 37 is provided. The temperature-switching-compartment discharge damper 37 is arranged in the lead-in air flow passage 15, and the temperature-switching-compartment blower 18 is arranged above the lead-in air flow passage 15. As the temperature-switching-compartment discharge damper 37 is opened and the temperature-switching-compartment blower 18 is driven, cool air flows from the cooler 11 via the lead-in air flow passage 15 into the temperature switching compartment 3. Based on the amount of opening and closing of the temperature-switching-compartment discharge damper 37, the amount of air flowing from the lead-in air flow passage 15 into the temperature switching compartment 3 is adjusted.

In a lower left part of the temperature switching compartment 3, a temperature-switching-compartment return damper 38 is provided. The temperature-switching-compartment return damper 38 opens and closes a return air flow passage 17 which extends downward, and the air inside the temperature switching compartment 3 returns via the return air flow passage 17 to the cool air passage 31.

The cooler 11 is formed of a serpentine refrigerant pipe 11a through which a refrigerant passes. Left and right end parts of the refrigerant pipe 11a are supported by an end plate 11b. The refrigerant pipe 11a is provided with, to be in contact therewith, a large number of fins (unillustrated) for dissipating heat.

The air flowing from the temperature switching compartment 3 through the return air flow passage 17 is returned to the cooler 11 through an outlet port 17a which is provided in the middle in the up/down direction of the cooler 11. The cool air flowing out from the freezer compartment 6 through the freezer-compartment return port 22 returns to a lower part of the cooler 11, and the cool air that flows out from the vegetable compartment 5 and flows through the return passage 46 (see Fig. 3) returns to below the cooler 11.

Accordingly, the cool air that has flowed out from different storage compartments is returned to the cooler 11 in a scattered manner. Thus, the frost due to the cool air, that has circulated through the different storage compartments and returned and that includes moisture, is prevented from concentrating on one part, but is scattered on the entire cooler 11. This helps prevent blocking, due to frost, of the flow of cool air, and hence prevent degraded cooling performance of the cooler 11.

The cool air that has flowed out from the freezer compartment 6 through the freezer-compartment return port 22 is led between the end plate 11b on either side. The cool air that has flowed out from the vegetable compartment 5 is led to the inner side and the outer side, i.e. to the entire left/right direction, of the end plate 11b on either side of the cooler 11 via the return air flow passage 46 (see Fig. 3).

Thus, the heat exchange area of the cool air flowed out from the vegetable compartment 5 is larger than that of the cool air flowed out from the freezer compartment 6. Accordingly, without cooling more than necessary the low-temperature cool air returning from the freezer compartment 6, the high-temperature cool air returning from the vegetable compartment 5 is cooled by the entire cooler 11, further enhancing the heat exchange efficiency of the cooler 11.

The temperature switching compartment 3 may be maintained at a freezing temperature, and thus the end plate 11b is provided with a cut-out part (unillustrated) in a place opposing the outlet port 17a of the return air flow passage 17. Thus, the cool air that has flowed out from the temperature switching compartment 3 is led between the end plate 11b on either side so as to scatter the cool air. Accordingly, condensation on the cooler 11 is scattered and blocking is further prevented.

In an upper part of the refrigerant pipe 11a, a gas-liquid separator 45 is connected. The gas-liquid separator 45 is arranged in an end part on the ice-making compartment 4 side away from the temperature switching compartment 3. Thus, even though the temperature-switching-compartment discharge damper 37 is arranged in a lower part of the temperature switching compartment 3, it does not interfere with the gas-liquid separator 45. As a result, it is possible to arrange the refrigerator-compartment damper 20 at the back of the vertical heat-insulating wall 36 while avoiding the interference between the refrigerator-compartment damper 20 and the temperature-switching-compartment discharge damper 37.

In the refrigerator structured as described above, the cool air generated by the cooler 11 is blown into the ice-making compartment 4 by driving of the freezer-compartment blower 12. The cool air blown into the ice-making compartment 4 flows through the ice-making compartment 4 and the freezing compartment 6, and flows out through the freezer-compartment return port 22 to return to the cooler 11. In this way, inside the ice-making compartment 4 and the freezer compartment 6 is cooled.

The cool air that has branched at the discharge side of the freezer-compartment blower 12 flows via the refrigerator-compartment damper 20 into the cool air passage 32 by driving of the refrigerator-compartment blower 23. The cool air blown out from the refrigerator-compartment blower 23 flows through the inlet portion 32c of the cool air passage 32 and includes ions generated by the ion generator 130.

As being narrowed down in the front/rear direction in the inlet portion 32c, the cool air including the ions flows and spreads out to the left and right in the widened portion 32d. This rapidly lowers the flow rate of the cool air and dynamic pressure is converted into static pressure. Lowered flow rate of the cool air improves the blowing efficiency. It also reduces disappearance of ions due to collision, further improving the sterilizing effect.

Part of the cool air flowing through the widened portion 32d is discharged through the discharge ports 103a and 103b into the chilled compartment 21. The cool air discharged through the discharge ports 103a and 103b flows into the case 107 in the chilled compartment 21 to cool and sterilize inside the case 107. The cool air flowed into the cool air passage 32 is immediately fed to the chilled compartment 21, and thus articles-to-be-stored in the case 107 is cooled at a temperature lower than that outside the chilled compartment 21.

The rest of the cool air flowing through the cool air passage 32 rises through the first and second branched passages 32a and 32b that branches to the right and left, respectively, and is discharged into the refrigerator compartment 2 through the discharge ports 104a, 104b, 105a, and 105b. In this way, inside the refrigerator compartment 2 is cooled and sterilized.

Cold of the cool air flowing through the cool air passage 32 is transmitted via the panel 100 to the member 101. In addition, the cool air discharged through the discharge ports 105a and 105b flows down along the member 101 and cold is transmitted to the member 101. Thus, even though the refrigerator compartment 2 is large, cold is released from a wide area of the rear face of the refrigerator compartment 2, and the temperature distribution of the refrigerator compartment 2 is made even. Furthermore, the member 101 covers the front of the space portion 111 provided between the first and second branched passages 32a and 32b, and thus cold of the cool air is released to a wider area. Accordingly, more even temperature distribution of the refrigerator compartment 2 is achieved.

The cool air that has been discharged into the refrigerator compartment 2 flows frontward above the storage shelves 41 and the partition plate 41a, and exchanges heat with articles-to-be-stored placed on those. Then, the cool air cools articles-to-be-stored in the storage pockets 42 provided at the door 2a and flows downward. The cool air flowing downward flows between a side wall - on the refrigerator compartment outlet port 2b side - of the refrigerator compartment 2 and the outer side of the case 107, and flows through the refrigerator-compartment outlet port 2b into the connection passage 34.

The cool air flowing through the connection passage 34 flows into the vegetable compartment 5. The cool air flowed into the vegetable compartment 5 flows therethrough to cool and sterilize inside the vegetable compartment 5. The cool air flowed through the vegetable compartment 5 returns to the cooler 11 via the return air flow passage 46. In this way, inside the refrigerator compartment 2 and the vegetable compartment 5 is cooled, and when temperature reaches a set temperature, the refrigerator-compartment damper 20 is closed. Ions included in the cool air that has returned to the cooler 11 flows via the freezer-compartment blower 12 into the ice-making compartment 4 and the freezer compartment 6 to sterilize them.

The cool air that has branched at the discharge side of the freezer-compartment blower 12 flows via the temperature-switching-compartment discharge damper 37 into the temperature switching compartment 3 by driving of the temperature-switching-compartment blower 18. The cool air flowed into the temperature switching compartment 3 flows therethrough, and flows out via the temperature-switching-compartment return damper 38 to return to the cooler 11 via the return air flow passage 17. In this way, inside the temperature switching compartment 3 is cooled and sterilized.

As described above, the temperature in the temperature switching compartment 3 can be changed by operation of the user. For the driving mode of the temperature switching compartment 3, different cooling modes are provided according to the temperature range, i.e., refrigeration (3 °C), chilled (- 1 °C), partial freezing (- 9 °C), and freezing (- 16 °C).

This permits the user to cool and preserve articles-to-be-stored by freezing or refrigerating them at a desired temperature. Switching of the temperature in the compartment can be performed by varying the amount of opening of the temperature-switching-compartment discharge damper 37. For example, on switching the temperature in the compartment from freezing to refrigeration, the heater 16 may be energized to raise the temperature. This permits the temperature in the compartment to be switched swiftly to a desired temperature.

By energizing the heater 16, it is possible to switch the temperature in the temperature switching compartment 3 from a low-temperature side, at which articles-to-be-stored are cooled and preserved, to a high-temperature side, at which the temperature is higher than normal temperature. This permits precooked heating foods to be kept warm temporary, to be cooked by warming, etc.

According to this embodiment, since the cool air passage 32 branches into the first and second branched passages 32a and 32b due to the space portion 111, it is possible to keep an adequate flow passage area of the cool air passage 32 even with a larger refrigerator 1 with increased left-right width. Thus, the cool air from the refrigerator-compartment blower 23 is blown sufficiently to an upper part of the refrigerator compartment 2. The cool air is also blown to an end part of the refrigerator compartment 2.

Since the ion generator 130 is provided in the inlet portion 32c at the upstream of where the cool air passage 32 branches into the first and second branched passages 32a and 32b, the cool air including ions flows, as it spreads out, along the rear face of the refrigerator compartment 2, and is discharged through the discharge ports 104a, 104b, 105a, and 105b. Accordingly, the ion concentration in the refrigerator compartment 2 is made even, and the sterilizing effect is improved.

Since the cool air passage 32 widens at the widened portion 32d at the downstream of where the cool air flows into the cool air passage 32, and the ion generator 130 is provided in the inlet portion 32c at the upstream of where the cool air passage 32 widens, the cool air includes ions before they are diffused. Accordingly, the cool air flowing through the cool air passage 32 surely includes ions, and the sterilizing effect is further improved.

The temperature of the cool air flowing into the cool air passage 32 rises above the temperature (for example, - 25 °C) of the cool air generated by the cooler 11, so as to be about - 18 °C. The temperature of the cool air in contact with the ion generator 130 is higher than - 20 °C, and thus frost accumulating on the electrode of the ion generator 130 is reduced.

The ion generator 130 is arranged in the inlet portion 32c with a high voltage generator, which applies a high voltage to the electrode, and the electrode unitized to be compact. Here, since the temperature of the cool air in contact with the ion generator 130 is higher than - 20 °C, electrical components in the high voltage generator is maintained higher than - 20 °C. This helps prevent failure of maintaining discharging due to the temperature of the electrical components in the high voltage generator falling to - 20 °C or below. Accordingly, the electrode surely discharges and generates ions.

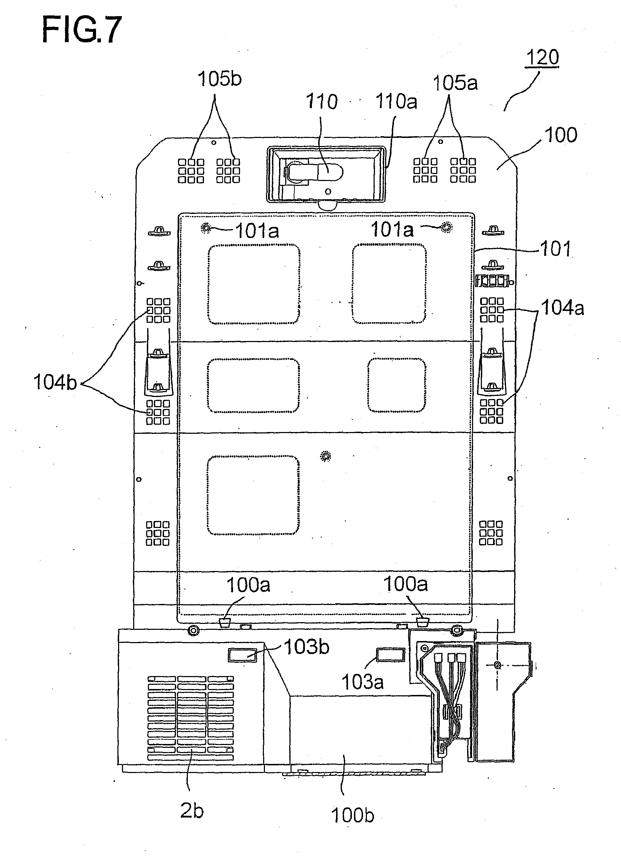

Next, a second embodiment of the invention will be described. Compared with the first embodiment shown in Figs. 1 to 6 described above, this embodiment differs in the configuration of a panel assembly. The rest of the parts are similar to those in the first embodiment. Fig. 7 is a front view showing a panel assembly 120 of a refrigerator according to this embodiment. For the sake of convenience of description, such parts as find their counterparts in Figs 1 to 6 described above are identified by common reference signs.

In the panel assembly 120, a member 101 having high thermal conductivity is arranged on the front face of a panel 100 comprising a resin molded component. For the panel 100, resin such as PP, PS, and ABS that is easy to process is used. For the member 101, metal such as aluminum and highly anticorrosive stainless steel is used.

In an upper part of the panel 100, a lamp housing 110a covered by a lamp cover (unillustrated) is recessed. Inside the lamp housing 110a, an illumination lamp 110 is arranged. The member 101 is arranged below the lamp housing 110a. Light emitted from the illumination lamp 110 illuminates inside a refrigerator compartment 2 from above, and is reflected at the surface of the member 101 to illuminate inside the refrigerator compartment 2 from behind.

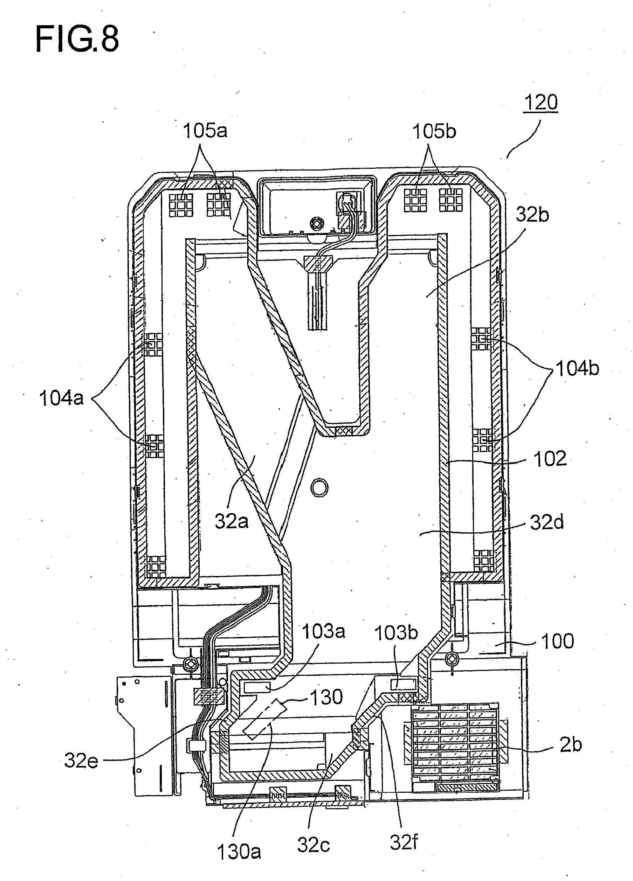

Around the member 101, discharge ports 104a, 104b, 105a, and 105b are open in the panel 100. The discharge ports 104a and 104b are arranged to sides of the member 101, and the discharge ports 105a and 105b are arranged above the member 101. In a place lopsidedly rightward in a lower part of the panel 100, a project portion 100b projecting frontward is provided. By the project portion 100b, an inlet portion 32c (see Fig. 8) into which cool air flows is formed.

Fig. 8 shows a rear view of the panel assembly 120. A sealing member 102 is affixed on a rib (unillustrated) that projects from the rear face of the panel 100, and thereby the outer shape of a cool air passage 32 is formed. The inlet portion 32c provided in a lower part of the panel assembly 120 is formed with a part thereof into which cool air flows located lopsidedly on the right side (the left side in Fig. 8) and with an upper part thereof inclined leftward. Thus, cold of the cool air is released via the cool air passage 32 to an ice-making compartment 4 at or below the ice point, and thus no cold is drawn by the temperature switching compartment 3, improving the cooling efficiency of the refrigerator 1.

In the inlet portion 32c, discharge ports 103a and 103b that discharge cool air to a chilled compartment 21 are open in the panel 100. At the downstream of the inlet portion 32c, a widened portion 32d where the flow passage widens is provided, and a first and second branched passages 32a and 32b that branch from the widened portion 32d are provided. The first branched passage 32a extends from the widened portion 32d to the right (to the left in Fig. 8) upward in an inclined manner, and bends at the top end to extend downward. The second branched passage 32d extends from the widened portion 32d vertically upward, and bends at the top end to extend downward.

The discharge ports 105a and 105b are provided at upper ends of the first and second branched passages 32a and 32b, respectively. The discharge ports 104a and 104b are provided in those parts of the first and second branched passages 32a and 32b which extend from the upper ends downward.

In the inlet portion 32c of the cool air passage 32, an ion generator 130 is arranged lopsidedly on the right side (the left side in Fig. 8). The ion generator 130 is arranged in an inclined manner with the longitudinal direction thereof aligned along the inlet portion 32c and with a downstream end thereof pointing to the widened portion 32d. An electrode face 130a of the ion generator 130 faces a side wall 32f of the inlet portion 32c, which is on the farther side, and a face thereof opposite from the electrode face 130a is arranged apart from a wall face 32e of the inlet portion 32c. Thus, the electrode face 130a is provided along the cool air rising through the inlet portion 32c, and the ion generator 130 has a function of a wind deflecting plate, permitting the cool air to be led to the widened portion 32d.

The cool air that has flowed into the inlet portion 32c includes ions generated by the ion generator 130. Part of the cool air including the ions is discharged through the discharge ports 103a and 103b to the chilled compartment 21. The cool air rises via the widened portion 32d through the first and second branched passages 32a and 32b. The cool air rising through the first and second branched passages 32a and 32b is discharged through the discharge ports 105a and 105b at the upper ends. Further, the cool air descends from the upper ends of the first and second branched passages 32a and 32b, and is discharged through the discharge ports 104a and 104b. In this way, the refrigerator compartment 2 is cooled and sterilized.

According to this embodiment, since the ion generator 130 is provided in the inlet portion 32c at the upstream of where the cool air passage 32 branches into the first and second branched passages 32a and 32b as in the first embodiment, the cool air including the ions flows, as it spreads out, along the rear face of the refrigerator compartment 2, and is discharged through the discharge ports 104a, 104b, 105a, and 105b. Accordingly, the ion concentration in the refrigerator compartment 2 is made even, and the sterilizing effect is improved.

Since the ion generator 130 is provided in the inlet portion 32c at the upstream of the widened portion 32d, the cool air includes the ions before they are diffused. Accordingly, the cool air flowing through the cool air passage 32 surely includes the ions, and the sterilizing effect is improved.

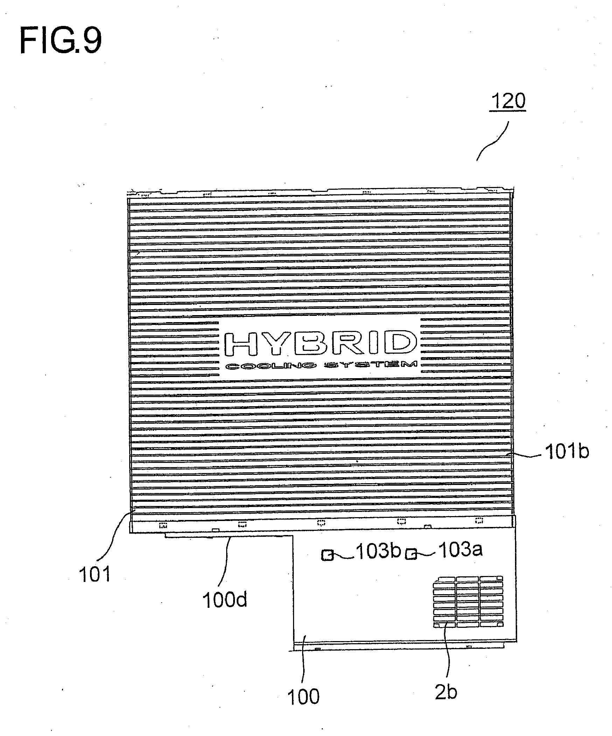

Next, a third embodiment of the present invention will be described. Compared with the first embodiment shown in Figs. 1 to 6 described above, this embodiment differs in the configuration of a panel assembly. The rest of the parts are similar to those in the first embodiment. Fig. 9 is a front view showing a panel assembly 120 of a refrigerator according to this embodiment. For the sake of convenience of description, such parts as find their counterparts in Figs 1 to 6 described above are identified by common reference signs.

In the panel assembly 120, a member 101 having high thermal conductivity is arranged on the front face of a panel 100 comprising a resin molded component. For the panel 100, resin such as PP, PS, and ABS that is easy to process is used. For the member 101, metal such as aluminum and highly anticorrosive stainless steel is used. At the surface of the member 101, elevation/depression portions 100b that extend horizontally by being bent are formed.

A lamp housing (unillustrated) that houses an illumination lamp 110 (see Fig. 5) is provided in an upper part of the panel assembly 120. Light emitted from the illumination lamp 110 illuminates inside a refrigerator compartment 2 from above, and is reflected at the elevation/depression portions 100b of the member 101 to illuminate inside the refrigerator compartment 2 from behind.

In a lower left part of the panel 100, a cut-out part 100d is formed, and cool air flows through a duct (unillustrated) which is arranged in the cut-out part 100d. Specifically, the part, into which the cool air flows, of the cool air passage 32 that passes along the rear face of the refrigerator compartment 2 is arranged lopsidedly leftward in the left/right direction. Thus, a refrigerator-compartment outlet port 2b is arranged on the right side of the panel 100. In addition, a project portion 100b (see Fig. 5), as in the first and second embodiments, is not provided in a lower part of the panel 100; a refrigerator-compartment blower 23 (see Fig. 4) is arranged below the cut-out part 100d.

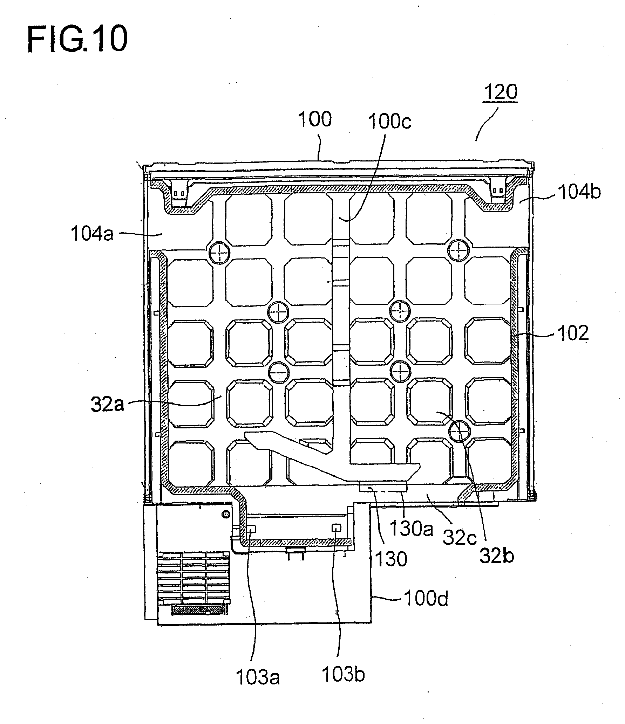

Fig. 10 shows a rear view of the panel assembly 120. A sealing member 102 is affixed on a rib (unillustrated) that projects from the rear face of the panel 100 so as to form the outer shape of the cool air passage 32. In the middle of the panel 100, a rib 100c with a substantially T shape is provided and, by the rib 100c, the first and second branched passages 32a and 32b are formed to branch. At the upper ends of the first and second passages 32a and 32b, discharge ports 104a and 104b that discharge the cool air sideward are provided.

In a lower part of the panel assembly 120, an inlet portion 32c into which the cool air flows is provided lopsidedly on the left side (the right side in Fig. 10) along the cut-out part 100d. In a lower part at a side of the inlet portion 32c, discharge ports 103a and 103b that discharge the cool air to the chilled compartment 21 are open in the panel 100.

In the inlet portion 32c, an ion generator 130 is arranged lopsidedly on the left side (the right side in Fig. 10) of the refrigerator compartment 2. The ion generator 130 is fitted to the lower face of the rib 100c with an electrode face 130a facing downward. Thus, the cool air flows into the rear face side of the panel assembly 120 via the inlet portion 32c, includes ions by making contact with the electrode face 130a, and then branches into the first and second branched passages 32a and 32b.

Part of the cool air including the ions is discharged through the discharge ports 103a and 103b into the chilled compartment 21. In addition, the cool air flows via the inlet portion 32c into the first and second branched passages 32a and 32b. Here, the air flow passage widens, and thus the flow rate is lowered, improving the blowing efficiency and reducing disappearance of ions. The cool air that has risen through the first and second branched passages 32a and 32b is discharged through the discharge ports 104a and 104b. In this way, the refrigerator compartment 2 is cooled and sterilized.

According to this embodiment, since the ion generator 130 is provided in the inlet portion 32c at the upstream of where the cool air passage 32 branches into the first and second branched passages 32a and 32b as in the first embodiment, the cool air including the ions flows, as it spreads out, along the rear face of the refrigerator compartment 2, and is discharged through the discharge ports 104a and 104b. Accordingly, the ion concentration in the refrigerator compartment 2 is made even, and the sterilizing effect is improved.

### Industrial Applicability

The present invention is applicable to refrigerators provided with an ion generator that generates ions.

## Claims

1. A refrigerator (1) comprising:
a storage compartment (2) cooling to preserve an article-to-be-stored;
a cooler (11) generating cool air;
a cool air passage (32) arranged at a rear face of the storage compartment (2) and through which cool air generated by the cooler (11) flows;
a discharge port (103a, 103b, 104a, 104b, 105a, 105b) opened in the cool air passage (32) and discharging cool air into the storage compartment (2); and
an ion generator (130) arranged in the cool air passage (32) and generating ions,
**characterized in that**
the cool air passage (32) includes
an inlet portion (32c) into which cold air from the cooler (11) flows along the rear face of the storage compartment (2),
a widened portion (32d) which is arranged downstream of the inlet portion (32c), in which a flow passage after cool air has flowed into the inlet portion (32c) is wider than in the inlet portion (32c), and which is arranged along the rear face of the storage compartment (2), and
first and second branched passages (32a, 32b) branching from the widened portion (32d) forming the flow passage after cool air has flowed into the inlet portion (32c),
the discharge port (103a, 103b, 104a, 104b, 105a, 105b) is arranged inside or downstream of the widened portion (32d), and
the ion generator (130) is arranged in the inlet portion (32c) arranged upstream of the widened portion (32d) from which the first and second branched passages (32a, 32b) branch.

2. The refrigerator according to claim 1,
wherein a part, into which cool air flows, of the cool air passage (32) is provided lopsidedly in one of left and right directions, and
wherein a longitudinal direction of the ion generator (130) is inclined such that a downstream end thereof points in another of the left and right directions.

3. The refrigerator according to claim 1,
wherein the ion generator (130) is arranged lopsidedly in a direction perpendicular to a direction in which cool air flows, with an electrode face (130a) that generates ions aligned along a direction in which cool air flows and with a face opposite from the electrode face (130a) being apart from a wall face of the cool air passage (32).

4. The refrigerator according to claim 1, configured so that the temperature of cool air that is in contact with the ion generator (130) is higher than - 20 °C.

5. The refrigerator according to claim 1,
wherein the storage compartment comprises a refrigerator compartment (2), the storage compartment being provided with a separated compartment (21) with a temperature lower than refrigeration temperature, and the discharge port (103a, 103b) is provided inside the widened portion (32d) to discharge cold air into the separated compartment (21), so that the ion generator (130) arranged in the inlet portion (32c) upstream of the widened portion (32d) is arranged upstream of the discharge port (103a, 103b) in the separated compartment (21).

## Patentansprüche

1. Kühlschrank (1), der Folgendes umfasst:
ein Aufbewahrungsfach (2), das kühlt, um einen aufzubewahrenden Artikel zu konservieren;
eine Kühlvorrichtung (11), die kühle Luft erzeugt;
einen Durchgang (32) für kühle Luft, der an einer Rückseite des Aufbewahrungsfachs (2) angeordnet ist, und durch den von der Kühlvorrichtung (11) erzeugte kühle Luft strömt;
eine Auslassöffnung (103a, 103b, 104a, 104b, 105a, 105b), die in dem Durchgang (32) für kühle Luft geöffnet ist und kühle Luft in das Aufbewahrungsfach (2) auslässt; und
einen Ionenerzeuger (130), der in dem Durchgang (32) für kühle Luft angeordnet ist und Ionen erzeugt,
**dadurch gekennzeichnet, dass**
der Durchgang (32) für kühle Luft Folgendes umfasst:
einen Einlassabschnitt (32c), in den kalte Luft von der Kühlvorrichtung (11) entlang der Rückseite des Aufbewahrungsfachs (2) strömt,
einen erweiterten Abschnitt (32d), der stromabwärts des Einlassabschnitts (32c) angeordnet ist, in dem ein Strömungsdurchgang, nachdem kühle Luft in den Einlassabschnitt (32c) geströmt ist, breiter ist als in dem Einlassabschnitt (32c), und der entlang der Rückseite des Aufbewahrungsfachs (2) angeordnet ist, und
einen ersten und einen zweiten Zweigdurchgang (32a, 32b), die von dem erweiterten Abschnitt (32d) abzweigen, die den Strömungsdurchgang bilden, nachdem kühle Luft in den Einlassabschnitt (32c) geströmt ist,
die Auslassöffnung (103a, 103b, 104a, 104b, 105a, 105b) in dem oder
stromabwärts des erweiterten Abschnitts (32d) angeordnet ist, und
der Ionenerzeuger (130) in dem stromaufwärts des erweiterten Abschnitts (32d), von dem der erste und der zweite Zweigdurchgang (32a, 32b) abzweigen, angeordneten Einlassabschnitt (32c) angeordnet ist.

2. Kühlschrank nach Anspruch 1,
wobei ein Teil, in den kühle Luft strömt, des Durchgangs (32) für kühle Luft in einer von der linken und der rechten Richtung schräg bereitgestellt ist, und
wobei eine Längsrichtung des Ionenerzeugers (130) derart geneigt ist, dass ein stromabwärtiges Ende desselben in eine andere von der linken und der rechten Richtung weist.

3. Kühlschrank nach Anspruch 1,
wobei der Ionenerzeuger (130) in einer zu einer Richtung, in der kühle Luft strömt, senkrechten Richtung schräg angeordnet ist, wobei eine Elektrodenfläche (130a), die Ionen erzeugt, entlang einer Richtung ausgerichtet ist, in der kühle Luft strömt, und wobei eine der Elektrodenfläche (130a) gegenüberliegende Fläche von einer Wandfläche des Durchgangs (32) für kühle Luft beabstandet ist.

4. Kühlschrank nach Anspruch 1, der derart konfiguriert ist, dass die Temperatur von kühler Luft, die sich mit dem Ionenerzeuger (130) in Kontakt befindet, höher als -20 °C ist.

5. Kühlschrank nach Anspruch 1,
wobei das Aufbewahrungsfach ein Kühlfach (2) umfasst, wobei das Aufbewahrungsfach mit einem abgetrennten Fach (21) mit einer Temperatur unter der Kühltemperatur versehen ist und die Auslassöffnung (103a, 103b) in dem erweiterten Abschnitt (32d) bereitgestellt ist, um kalte Luft in das abgetrennte Fach (21) auszulassen, sodass der in dem Einlassabschnitt (32c) stromaufwärts des erweiterten Abschnitts (32d) angeordnete Ionenerzeuger (130) stromaufwärts der Auslassöffnung (103a, 103b) in dem abgetrennten Fach (21) angeordnet ist.

## Revendications

1. Réfrigérateur (1) comportant :
un compartiment de stockage (2) servant à des fins de refroidissement pour préserver un article destiné à être stocké ;
un refroidisseur (11) servant à générer de l'air froid ;
un passage d'air froid (32) agencé au niveau d'une face arrière du compartiment de stockage (2) et au travers duquel l'air froid généré par le refroidisseur (11) s'écoule ;
un orifice de décharge (103a, 103b, 104a, 104b, 105a, 105b) ouvert dans le passage d'air froid (32) et déchargeant de l'air froid dans le compartiment de stockage (2) ; et
un générateur d'ions (130) agencé dans le passage d'air froid (32) et générant des ions,
**caractérisé en ce que**
le passage d'air froid (32) comprend
une partie d'entrée (32c) dans laquelle l'air froid en provenance du refroidisseur (11) s'écoule le long de la face arrière du compartiment de stockage (2),
une partie élargie (32d) qui est agencée en aval par rapport à la partie d'entrée (32c), dans laquelle un passage d'écoulement après que l'air froid s'est écoulé dans la partie d'entrée (32c) est plus large que dans la partie d'entrée (32c), et qui est agencée le long de la face arrière du compartiment de stockage (2), et
des premier et deuxième passages ramifiés (32a, 32b) se ramifiant en provenance de la partie élargie (32d) formant le passage d'écoulement après que l'air froid s'est écoulé dans la partie d'entrée (32c),
l'orifice de décharge (103a, 103b, 104a, 104b, 105a, 105b) est agencé à l'intérieur de la partie élargie (32d) ou en aval par rapport à celle-ci, et
le générateur d'ions (130) est agencé dans la partie d'entrée (32c) agencée en amont par rapport à la partie élargie (32d) en provenance de laquelle les premier et deuxième passages ramifiés (32a, 32b) se ramifient.

2. Réfrigérateur selon la revendication 1,
dans lequel une partie, dans laquelle l'air froid s'écoule, du passage d'air froid (32) est mise en œuvre de travers dans l'une des directions allant vers la gauche et allant vers la droite, et
dans lequel une direction allant dans le sens longitudinal du générateur d'ions (130) est inclinée de telle sorte qu'une extrémité en aval de celui-ci pointe dans une autre des directions allant vers la gauche et allant vers la droite.

3. Réfrigérateur selon la revendication 1,
dans lequel le générateur d'ions (130) est agencé de travers dans une direction perpendiculaire par rapport à une direction dans laquelle l'air froid s'écoule, avec une face d'électrode (130a) qui génère des ions alignée le long d'une direction dans laquelle l'air froid s'écoule et avec une face opposée par rapport à la face d'électrode (130a) selon un espacement par rapport à une face de paroi du passage d'air froid (32).

4. Réfrigérateur selon la revendication 1,
configuré de telle sorte que la température de l'air froid qui est en contact avec le générateur d'ions (130) est supérieure à -20 °C.

5. Réfrigérateur selon la revendication 1,
dans lequel le compartiment de stockage comporte un compartiment de réfrigérateur (2), le compartiment de stockage comportant un compartiment séparé (21) avec une température inférieure à la température de réfrigération, et l'orifice de décharge (103a, 103b) est mis en œuvre à l'intérieur de la partie élargie (32d) pour décharger de l'air froid dans le compartiment séparé (21), de telle sorte que le générateur d'ions (130) agencé dans la partie d'entrée (32c) en amont par rapport à la partie élargie (32d) est agencé en amont par rapport à l'orifice de décharge (103a, 103b) dans le compartiment séparé (21).
